# EUROPÄISCHE PATENTSCHRIFT

(11) **EP 2 571 618 B1**
(45) Veröffentlichungstag und Bekanntmachung des Hinweises auf die Patenterteilung: **15.01.2014**
(21) Anmeldenummer: 11770349.6
(22) Anmeldetag: 04.10.2011
(51) Int. Cl.: B01L 3/00, C12M 1/22

(54) **VERSCHLUSS FÜR EINEN NÄHRMEDIENBEHÄLTER**
CLOSURE FOR A NUTRIENT MEDIUM CONTAINER
FERMETURE POUR RÉCIPIENT À PRODUIT ALIMENTAIRE

(30) Priorität: 23.11.2010 DE 102010052030
(43) Veröffentlichungstag der Anmeldung: 27.03.2013
(73) Patentinhaber: Sartorius Stedim Biotech GmbH, 37079 Göttingen (DE)
(72) Erfinder: PFLANZ, Karl, 37130 Gleichen (DE); GRAUS, Andreas, 37176 Nörten-Hardenberg (DE)
(74) Vertreter: Schneider, Peter Christian
(86) Internationale Anmeldenummer: PCT/EP2011/004923
(87) Internationale Veröffentlichungsnummer: WO 2012/069106

(56) Entgegenhaltungen:
- EP-A2- 0 171 174
- US-A- 3 158 553
- US-A- 3 339 770
- US-A- 3 474 004

## Beschreibung

### Gebiet der Erfindung

Die Erfindung betrifft einen Verschluss, insbesondere für einen Nährmedienbehälter, bei dem ein Unterteil durch ein Oberteil abdeckbar ist,
wobei das Unterteil mit dem Oberteil über mindestens eine Rastaufnahme und mindestens ein Rastteil durch Verdrehen um eine Längsachse von Unterteil und Oberteil gegeneinander verrastbar ist, und
wobei die Rastaufnahme eine von einer Schulter abgedeckte quer zur Längsachse verlaufende Aufnahmenut aufweist, in die das Rastteil eingreift.

### Stand der Technik

Aus der DE 10 2007 027 273 A1 ist ein Verschluss für einen Nährmedienbehälter bekannt, bei dem ein Unterteil durch ein Oberteil abdeckbar ist. Das Unterteil und das Oberteil sind über zwei oder mehr Rastaufnahmen und zwei oder mehr Rastteile durch Verdrehen um eine Längsachse gegeneinander verrastbar. Dazu weist eine Rastaufnahme eine von einer Schulter abgedeckte quer zur Längsachse verlaufende Aufnahmenut auf, in die das Rastteil eingreift.

Nachteilig dabei ist, dass bei Verwendung in automatischen Systemen, bei denen das Oberteil automatisiert auf das Unterteil aufgesetzt und/oder abgenommen wird, beispielsweise durch einen Roboterarm, eine unerwünschte Fehlerrate durch Fehlaufsetzungen auftritt, wenn beim Aufsetzen das Rastteil auf die Schultern der Rastaufnahme auftrifft.

Die gleichen Nachteile entstehen bei den aus der US 3,158,553 A, US 2006/0240549 A1, US 6,969,606 B2 und US 6,969,607 B2 bekannten Verschlüssen. Aus diesen Druckschriften sind ebenfalls Nährmittelbehälter bzw. sogenannte Petrischalen bekannt, bei denen ein Unterteil eine Rastaufnahme mit einer quer zur Längs- bzw. Drehachse verlaufenden Schulter aufweist, die in einem verrasteten Zustand ein Rastteil eines Oberteils bzw. Deckels abdeckt.

Auch diese bekannten Verschlüsse führen zu unerwünschten Fehlaufsetzungen in automatischen Anlagen.

### Aufgabenstellung

Aufgabe der vorliegenden Erfindung ist es daher, die bekannten Verschlüsse bzw. Verriegelungen, insbesondere an Nährmittelbehältern, so auszubilden, dass die Zahl von Fehlaufsetzungen beim automatisierten Aufsetzen des Oberteils auf das Unterteil verringert bzw. vermieden wird.

### Darstellung der Erfindung

Diese Aufgabe wird in Verbindung mit dem Oberbegriff des Anspruches 1 dadurch gelöst, dass die Schulter auf ihrer der Aufnahmenut abgewandten Außenseite mindestens eine zur Längsachse schräg verlaufende Auflauffläche aufweist.

Durch die nach einer Seite hin schräg verlaufende Schulter bzw. deren Auflauffläche verdrehen sich beim Auftreffen des Rastteiles auf die Schulter der Rastaufnahme Ober- und Unterteil gegeneinander, sodass es zuverlässig zu keinen Fehlaufsetzungen kommt.

Gemäß der Erfindung weist die Außenseite der Schulter zwei zur Längsachse schräg verlaufende, sich giebelförmig treffende Auflaufflächen auf.

Durch die giebelförmig ausgebildete Schulter wird bei gleicher Schulterbreite der Verdrehwinkel des Oberteils gegenüber dem Unterteil halbiert.

Bei einer weiteren bevorzugten Ausführungsform der Erfindung weist die Aufnahmenut der Rastaufnahme an ihrem Ende einen Anschlag für das Rastteil auf. Dadurch wird sichergestellt, dass Unterteil und Oberteil immer in der gleichen Rastposition ineinander verrasten.

Gemäß einer weiteren bevorzugten Ausführungsform der Erfindung ist die Aufnahmenut an ihrem dem Anschlag abgewandten Ende von einem Nuteingang begrenzt, der von dem Anfang der Schulter festgelegt wird, wobei dem Nuteingang eine von dem Rastteil zu überwindende Schwelle vorgelagert ist.

Eine solche Schwelle hat den Vorteil, dass das Oberteil nicht versehentlich, sondern erst durch gezielte Krafteinwirkung mit dem Unterteil verrasten kann. Die Schwelle ist dabei bevorzugt als ein vorgelagerter Viertel- oder Halbzylinder ausgebildet.

Eine versehentliche Verrastung des Oberteils mit dem Unterteil ist insbesondere im Zuge einer Fehlaufsetzung durch einen Roboterarm unerwünscht, wenn eine Vielzahl von Oberund Unterteilen in jeweils paarweise lose aufgelegtem Zustand auf einem laufenden Förderband platziert sind. In diesem Fall würde der Roboterarm nach versehentlicher Verrastung des Oberteils mit dem Unterteil und vor Befüllung des Unterteils mit einem Medium, insbesondere mit einem Nährmedium, nicht nur, wie erwünscht, das Oberteil von dem Unterteil für die nachfolgende Befüllung mit dem Medium abheben, sondern vielmehr würde der gesamte Behälter aus miteinander verrastetem Ober- und Unterteil durch den Roboterarm von dem Förderband abgehoben. Das Nährmedium würde in unerwünschter Weise das laufende Förderband kontaminieren, weil es nicht in das Unterteil, sondern auf das Förderband dosiert würde.

Dieser Vorteil einer Vermeidung einer versehentlichen Verrastung des Oberteils mit dem Unterteil ist besonders zuverlässig realisierbar, wenn die vom Rastteil zu überwindende Schwelle am Nuteingang der Aufnahmenut in Kombination mit den beiden auf der Außenseite der Schulter der Rastaufnahme schräg verlaufenden, sich giebelförmig treffenden Auflaufflächen vorliegt, weil beim Ablegen des Oberteils auf dem Unterteil durch den Roboterarm das Rastteil mit ungefähr gleicher Wahrscheinlichkeit an einer der beiden giebelförmigen Auflaufflächen hinabgleitet und links oder rechts von der Aufnahmenut zu liegen kommt, ohne mit der Aufnahmenut zu verrasten.

Nach einer weiteren bevorzugten Ausführungsform der Erfindung sind die Rastaufnahme an dem Unterteil und das Rastteil an dem Oberteil angeordnet. Grundsätzlich ist es aber auch möglich, die Rastaufnahme an dem Oberteil und das Rastteil an dem Unterteil anzuordnen.

Gemäß einer weiteren bevorzugten Ausführungsform der Erfindung ist die Rastaufnahme dem Boden des Unterteils benachbart in radialer Richtung außen an einer Außenwand des Unterteils angeordnet, wobei die Aufnahmenut in radialer Richtung nach außen offen ist.

Entsprechend ist dann das Rastteil in radialer Richtung nach innen an einer die Außenwand des Unterteils übergreifenden äußeren Oberteilwand des Oberteils angeordnet. Das Rastteil ist einer einem Deckel des Oberteils abgewandten freien Stirnfläche der äußeren Oberteilwand benachbart angeordnet.

Nach einer weiteren bevorzugten Ausführungsform der Erfindung ist das Rastteil als ein auf die Aufnahmenut abgestimmter Fixierzapfen ausgebildet. Die Kreisform des Fixierzapfens erlaubt ein reibungsarmes Gleiten auf den Auflageflächen.

Gemäß einer weiteren bevorzugten Ausführungsform der Erfindung sind das Unterteil und das Oberteil über zwei oder mehr äquidistant zueinander angeordnete Rastaufnahmen und Rastteile miteinander verrastbar. Die Zahl der Fehlaufsetzungen ist erfindungsgemäß nicht mehr abhängig von der Zahl der Rastaufnahmen.

Die äquidistant am Umfang der Wände beabstandeten Rastaufnahmen und Rastteile ermöglichen ein gleichmäßiges Fixieren der Teile gegeneinander.

Weitere Einzelheiten der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibung der Zeichnungen

In den Zeichnungen zeigen:

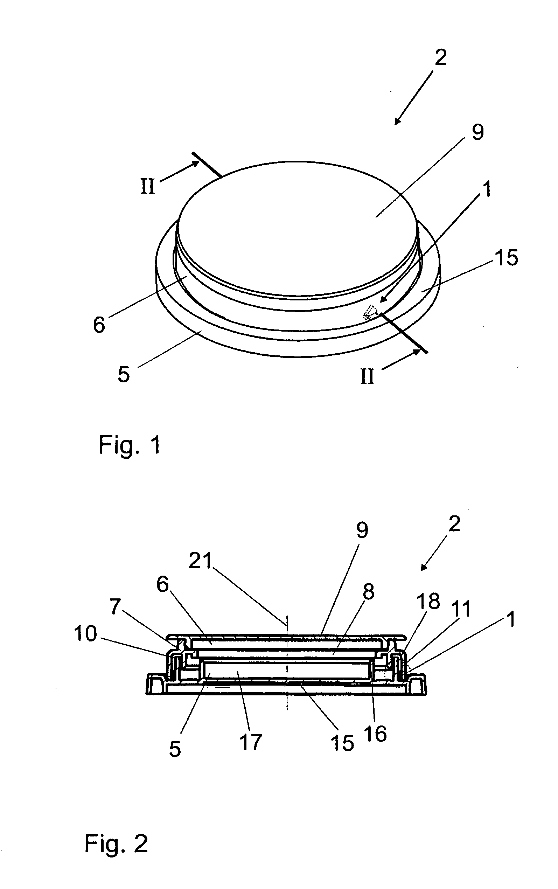

- Figur 1:: eine räumliche Darstellung eines Nährmedienbehälters mit einem gestrichelt dargestellten Verschluss,
- Figur 2:: eine Seitenansicht im Schnitt des Nährmedienbehälters von Figur 1 entlang der Linie II-II geschnitten,

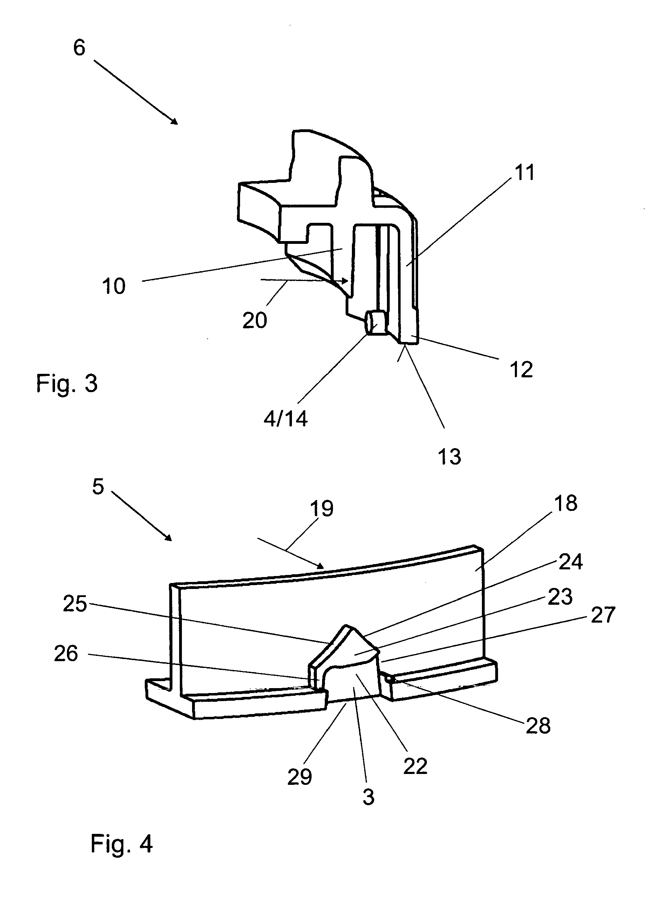

- Figur 3:: eine räumliche Darstellung eines Ausrisses des Oberteils von Figur 1 im Bereich des Rastteiles in vergrößerter Darstellung,
- Figur 4:: eine räumliche Darstellung eines Ausrisses des Unterteils von Figur 1 im Bereich der Rastaufnahme in vergrößerter Darstellung,

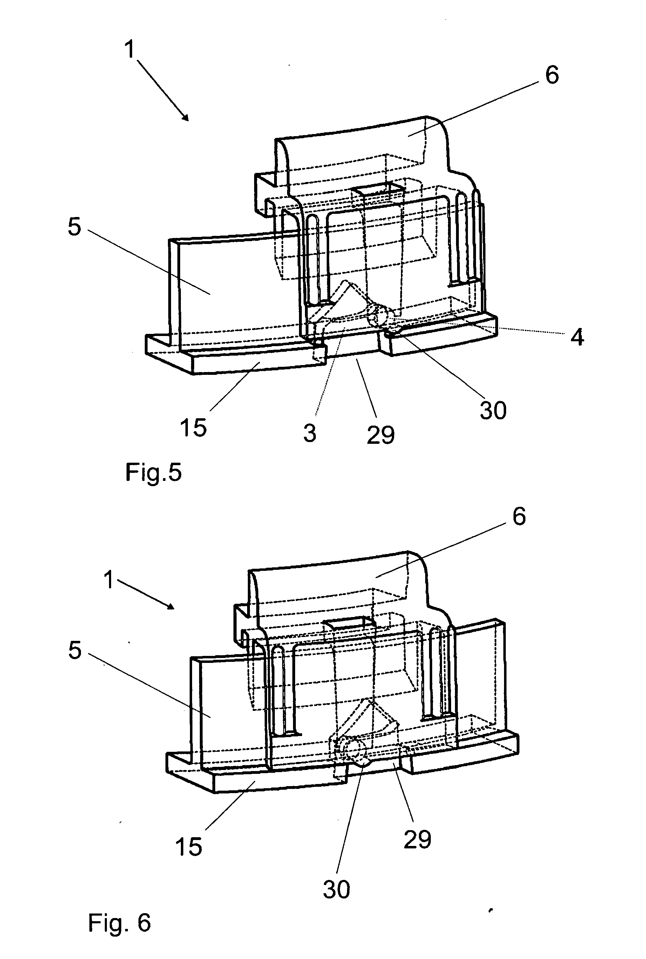

- Figur 5:: eine räumliche Darstellung des Unterteils von Figur 4 mit aufgesetztem Oberteil von Figur 3 in nicht verrasteter Stellung und
- Figur 6:: eine räumliche Darstellung im Ausriss des Oberteils und Unterteils von Figur 5 in verrasteter Stellung.

### Beschreibung der Ausführungsbeispiele

Ein Verschluss 1 eines Nährmedienbehälters 2 besteht im Wesentlichen aus einer Rastaufnahme 3 und einem Rastteil 4.

Der Nährmedienbehälter 2 besteht aus einem Unterteil 5 und einem Oberteil 6. Das Oberteil 6 im Ausführungsbeispiel ist zweiteilig ausgebildet und besteht aus einem Verbindungsring 7, dessen obere Öffnung 8 über eine Klemmverbindung von einem Deckel 9 verschlossen ist. Das Oberteil 6 weist einen von dem Deckel 9 weg gerichteten ringförmigen Bund 10 auf, der von einer zu ihm beabstandeten äußeren Oberteilwand 11 umgeben ist. Das dem Deckel 9 abgewandte freie Ende 12 der Oberteilwand 11 weist eine Stirnfläche 13 auf. An dem freien Ende 12 der äußeren Oberteilwand 11 sind benachbart zur Stirnfläche 13 einander gegenüberliegend zwei radial nach innen gerichtete Rastteile 4 angeordnet, die als Fixierzapfen 14 ausgebildet sind.

Das Unterteil 5, weist einen Boden 15 mit einer ringförmigen Innenwandung 16 auf, die einen Schalenraum 17 zur Aufnahme von Medien, insbesondere Nährmedien für die Mikrobiologie, begrenzt. Koaxial zur Innenwandung 16 weist das Unterteil 5 eine Außenwandung 18 auf, deren Innendurchmesser 19 auf den mit ihm korrespondierenden Außendurchmesser 20 des Bundes 10 des Oberteiles 6 abgestimmt ist. Das Oberteil 6 wird damit von seinem Außendurchmesser 20 des Bundes 10 in dem Innendurchmesser 19 der Außenwandung 18 des Unterteils 5 geführt. Die äußere Oberteilwand 11 des Oberteils 6 übergreift mit ihren Fixierzapfen 14 die Außenwandung 18 des Unterteils 5. An der Außenwandung 18 des Unterteils 5 ist dem Boden 15 benachbart die Rastaufnahme 3 angeordnet. Die Rastaufnahme 3 weist eine quer zur Längsachse 21, die orthogonal und mittig zum Boden 15 ausgebildet ist, eine querverlaufende Aufnahmenut 22 auf. Die Aufnahmenut 22 weist zum Oberteil 6 hin eine sie abdeckende oder begrenzende Schulter 23 auf. An ihrer der Aufnahmenut 22 abgewandten Außenseite weist die Schulter 23 zwei zur Längsachse 21 schräg verlaufende, sich giebelförmig treffende Auflaufflächen 24, 25 auf. Die Aufnahmenut 22 wird an einem Ende durch einen Anschlag 26 begrenzt, der sich zwischen Boden 15 und Schulter 23 erstreckt. An ihrem dem Anschlag 26 abgewandten Ende weist die Aufnahmenut 22 einen Nuteingang 27 auf. Dem Nuteingang 27 ist eine Schwelle 28 vorgelagert. Im Ausführungsbeispiel ist die Schwelle 28 als ein Viertelzylinder ausgebildet. Die Schwelle kann aber auch als Halbzylinder oder in jeder anderen Geometrie ausgebildet sein, die einen durch den Fixierzapfen 14 zu überwindenden Widerstand beim Ein- und Ausführen des Fixierzapfens 14 in die Aufnahmenut 22 darstellt.

Oberhalb der Schwelle 28 weist die Aufnahmenut 22 eine entsprechende Erweiterung auf.

Zum Boden 15 hin geht die Aufnahmenut 22 in eine Bodenaussparung 29 über. In die Bodenaussparung kann im verrasteten Zustand der Fixierzapfen 14 mit einem über die Stirnfläche 13 der äußeren Oberteilwand 11 hinausragenden Zylinderabschnitt hineinragen.

Die Oberteile 6 können automatisch gesteuert von einem Roboter bzw. von einer Handhabungseinheit auf das Unterteil 5 aufgesetzt werden. Soweit bei dem Aufsetzen das Rastteil 4 auf die Schulter 23 trifft, setzt der Fixierzapfen 14 auf eine der Auflaufflächen 24, 25 auf, wobei die Teile 5, 6 sich gegeneinander verdrehen, sodass der Fixierzapfen 14 seitlich neben der Schulter 23 bzw. der Rastaufnahme 3 auf den Boden 15 aufsetzt.

Entsprechend kann das Oberteil von einem Handhabungselement einfach abgenommen werden. Nach dem Befüllen des Nährmedienbehälters 2 wird durch Verdrehen des Oberteils 6 gegenüber dem Unterteil 5 der Fixierzapfen 14 über die Schwelle 28 durch den Nuteingang 27 in die Aufnahmenut 21 durch Verdrehen um die Längsachse 21 eingeführt.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum an Variationsmöglichkeiten an die Hand gegeben.

### Bezugszeichenliste

- 1: Verschluss
- 2: Nährmedienbehälter
- 3: Rastaufnahme
- 4: Rastteil
- 5: Unterteil von 2
- 6: Oberteil
- 7: Verbindungsring von 6
- 8: obere Öffnung von 7
- 9: Deckel von 6
- 10: Bund von 6
- 11: äußere Oberteilwand von 6
- 12: freies Ende von 11
- 13: Stirnfläche von 12
- 14: Fixierzapfen
- 15: Boden von 5
- 16: Innenwandung von 5
- 17: Schalenraum von 5
- 18: Außenwandung von 5
- 19: Innendurchmesser von 18
- 20: Außendurchmesser von 10
- 21: Längsachse
- 22: Aufnahmenut
- 23: Schulter
- 24: Auflauffläche, erste
- 25: Auflauffläche, zweite
- 26: Anschlag von 3
- 27: Nuteingang von 22
- 28: Schwelle
- 29: Bodenaussparung
- 30: Zylinderabschnitt von 14

## Patentansprüche

1. Verschluss, insbesondere für einen Nährmedienbehälter (2), bei dem ein Unterteil (5) durch ein Oberteil (6) abdeckbar ist,
wobei das Unterteil (5) und das Oberteil (6) über mindestens eine Rastaufnahme (3) und mindestens ein Rastteil (4) durch Verdrehen um eine Längsachse (21) von Unterteil (5) und Oberteil (6) gegeneinander verrastbar sind, und
wobei die Rastaufnahme (3) eine von einer Schulter (23) abgedeckte quer zur Längsachse (21) verlaufende Aufnahmenut (22) aufweist, in die das Rastteil (4) eingreift,
**dadurch gekennzeichnet,**
**dass** die Schulter (23) auf ihrer der Aufnahmenut (22) abgewandten Außenseite zwei zur Längsachse (21) schräg verlaufende, sich giebelförmig treffende Auflaufflächen (24, 25) aufweist.

2. Verschluss nach Anspruch 1,
**dadurch gekennzeichnet,**
**dass** die Aufnahmenut (22) der Rastaufnahme (3) an ihrem Ende einen Anschlag (26) für das Rastteil (4) aufweist.

3. Verschluss nach Anspruch 2,
**dadurch gekennzeichnet,**
**dass** die Aufnahmenut (22) an ihrem dem Anschlag (26) abgewandten Ende von einem Nuteingang (27) begrenzt ist, dem eine von dem Rastteil (4) zu überwindende Schwelle (28) vorgelagert ist.

4. Verschluss nach Anspruch 3,
**dadurch gekennzeichnet,**
**dass** die Schwelle (28) als ein vorgelagerter Viertel- oder Halbzylinder ausgebildet ist.

5. Verschluss nach einem der Ansprüche 1 bis 4,
**dadurch gekennzeichnet,**
**dass** die Rastaufnahme (3) an dem Unterteil (5) und das Rastteil (4) an dem Oberteil (6) angeordnet sind.

6. Verschluss nach Anspruch 4 oder 5,
**dadurch gekennzeichnet,**
**dass** die Rastaufnahme (3) dem Boden (15) des Unterteils (5) benachbart in radialer Richtung außen an einer Außenwand (18) des Unterteils (5) angeordnet ist, und dass die Aufnahmenut (22) in radialer Richtung nach Außen offen ist.

7. Verschluss nach einem der Ansprüche 4 bis 6,
**dadurch gekennzeichnet,**
**dass** das Rastteil (4) in radialer Richtung nach innen an einer die Außenwand (18) des Unterteils (5) übergreifenden Oberteilwand (11) benachbart einer einem Deckel (9) des Oberteils (6) abgewandten freien Stirnfläche (13) der äußeren Oberteilwand (11) angeordnet ist.

8. Verschluss nach einem der Ansprüche 1 bis 7,
**dadurch gekennzeichnet,**
**dass** das Rastteil (4) als ein auf die Aufnahmenut (22) abgestimmter Fixierzapfen (14) ausgebildet ist.

9. Verschluss nach einem der Ansprüche 1 bis 8,
**dadurch gekennzeichnet,**
**dass** das Unterteil (5) und das Oberteil (6) über zwei oder mehr äquidistant zueinander angeordnete Rastaufnahmen (3) und Rastteile (4) miteinander verrastbar sind.

## Claims

1. Closure, in particular for a nutrient medium container (2), wherein a lower part (5) can be covered by an upper part (6),
wherein the lower part (5) and the upper part (6) can be locked against each other by means of at least one locking receptacle (3) and at least one locking part (4) by rotating the lower part (5) and the upper part (6) about a longitudinal axis (21), and
wherein the locking receptacle (3) has a receptacle groove (22) that is covered by a shoulder (23) and that extends perpendicularly to the longitudinal axis (21), in which receptacle groove (22) the locking part (4) engages,
**characterized in that**
the shoulder (23) has on its exterior side facing away from the receptacle groove (22) two ramp surfaces (24, 25) that extend at an angle from the longitudinal axis (21) and meet to form a triangle shape.

2. Closure according to Claim 1,
**characterized in that**
the receptacle groove (22) of the locking receptacle (3) has at its end a contact surface (26) for the locking part (4).

3. Closure according to Claim 2,
**characterized in that**
the receptacle groove (22) is bordered at the end opposite to the contact surface (26) by a groove inlet (27) that is placed anterior to a threshold (28) that must be passed by the locking part (4).

4. Closure according to Claim 3,
**characterized in that**
the threshold (28) is arranged in an anterior position as a quarter-cylinder or half-cylinder.

5. Closure according to any of Claims 1 to 4,
**characterized in that**
the locking receptacle (3) is arranged on the lower part (5) and the locking part (4) is arranged on the upper part (6).

6. Closure according to Claim 4 or 5,
**characterized in that**
the locking receptacle (3) is arranged adjacent to the base (15) of the lower part (5), in a radial direction on the outside on an exterior wall (18) of the lower part (5) and that the receptacle groove (22) opens towards the exterior in a radial direction.

7. Closure according to any of Claims 4 to 6,
**characterized in that**
the locking part (4) is arranged in a radially inward direction on an upper part wall (11) that extends beyond the exterior wall (18) of the lower part (5), adjacent to a free front face (13) of the exterior upper part wall (11) that faces away from a lid (9) of the upper part (6).

8. Closure according to any of Claims 1 to 7,
**characterized in that**,
the locking part (4) is designed as a locking pin (14) which is made to fit the receptacle groove (22).

9. Closure according to any of Claims 1 to 8,
**characterized in that**
the lower part (5) and the upper part (6) can be locked against each other via two or more locking receptacles (3) and locking parts (4) arranged equidistantly with respect to one another.

## Revendications

1. Une fermeture, en particulier pour un récipient à produit alimentaire (2), présentant une partie inférieure (5) pouvant être recouverte par une partie supérieure (6),
la partie inférieure (5) et la partie supérieure (6) pouvant se bloquer l'une sur l'autre par l'intermédiaire d'au moins un logement d'encliquetage (3) et d'au moins un élément d'encliquetage (4), par la rotation de la partie inférieure (5) et de la partie supérieure (6) autour d'un axe longitudinal (21), et
le logement d'encliquetage (3) comportant une rainure de réception (22) s'étendant transversalement à l'axe longitudinal (21), qui est recouverte par un épaulement (23) et dans laquelle vient se loger l'élément d'encliquetage (4),
**caractérisée en ce que**,
l'épaulement (23) présente sur son côté externe opposé à la rainure de réception (22) deux surfaces de glissement (24, 25) qui s'étendent en formant un angle avec l'axe longitudinal (21) et se rencontrent tel un pignon.

2. Fermeture selon la revendication 1,
**caractérisée en ce que**,
la rainure de réception (22) du logement d'encliquetage (3) présente sur son extrémité une surface de contact (26) pour l'élément d'encliquetage (4).

3. Fermeture selon la revendication 2,
**caractérisée en ce que**,
la rainure de réception (22) est limitée à l'extrémité opposée à la surface de contact (26) par une entrée de rainure (27) placée dans une position antérieure au seuil (28) qui doit être traversé par l'élément d'encliquetage (4).

4. Fermeture selon la revendication 3,
**caractérisée en ce que**,
le seuil (28) est disposé dans une position antérieure sous forme d'un quart de cylindre ou d'un demi-cylindre.

5. Fermeture selon l'une quelconque des revendications 1 à 4,
**caractérisée en ce que**,
le logement d'encliquetage (3) est disposé sur la partie inférieure (5) et l'élément d'encliquetage (4) est disposé sur la partie supérieure.

6. Fermeture selon l'une quelconque des revendications 4 ou 5,
**caractérisée en ce que**,
le logement d'encliquetage (3) est disposé adjacent à la base (15) de la partie inférieure (5), dans une direction radiale à l'extérieur d'une paroi externe (18) de la partie inférieure (5) et **en ce que** la rainure de réception (22) s'ouvre vers l'extérieur dans une direction radiale.

7. Fermeture selon l'une quelconque des revendications 4 à 6,
**caractérisée en ce que**,
l'élément d'encliquetage (4) est disposé dans une direction radialement vers l'intérieur sur une paroi de la partie supérieure (11) qui s'étend au-delà de la paroi externe (18) de la partie inférieure (5), adjacente à une face avant libre (13) de la paroi de la partie supérieure externe (11) opposée à un couvercle (9) de la partie supérieure (6).

8. Fermeture selon l'une quelconque des revendications 1 à 7,
**caractérisée en ce que**,
l'élément d'encliquetage (4) est conçu comme une broche d'encliquetage (14) destinée à se loger dans la rainure de réception (22).

9. Fermeture selon l'une quelconque des revendications 1 à 8,
**caractérisée en ce que**,
la partie inférieure (5) et la partie supérieure (6) peuvent se bloquer l'une sur l'autre par l'intermédiaire de deux ou plusieurs logements d'encliquetage (3) et éléments d'encliquetage (4) disposés de façon équidistante l'un par rapport à l'autre.
